(19) Europäisches Patentamt
European Patent Office
Office européen des brevets

(11) **EP 2 758 093 B1**

(12) **EUROPEAN PATENT SPECIFICATION**

(45) Date of publication and mention
of the grant of the patent:
**21.12.2016 Bulletin 2016/51**

(51) Int Cl.:
*A61M 1/16* *(2006.01)*       *B01D 53/22* *(2006.01)*
*B01D 69/02* *(2006.01)*      *B01D 69/08* *(2006.01)*
*B82Y 30/00* *(2011.01)*      *B82Y 40/00* *(2011.01)*

(21) Application number: **12778132.6**

(22) Date of filing: **21.09.2012**

(86) International application number:
**PCT/IB2012/001856**

(87) International publication number:
**WO 2013/041950 (28.03.2013 Gazette 2013/13)**

(54) **GAS EXCHANGER COMPRISING NANOTUBES AND ARTIFICIAL LUNG**

GASAUSTAUSCHER MIT NANORÖHREN UND KÜNSTLICHE LUNGE

ÉCHANGEUR DE GAZ COMPRENANT DES NANOTUBES ET POUMON ARTIFICIEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.09.2011 US 201161538417 P**

(43) Date of publication of application:
**30.07.2014 Bulletin 2014/31**

(73) Proprietor: **Palti, Yoram**
**Haifa 34404 (IL)**

(72) Inventor: **Palti, Yoram**
**Haifa 34404 (IL)**

(74) Representative: **Boden, Keith McMurray**
**Fry Heath & Spence LLP**
**The Gables**
**Massetts Road**
**Horley**
**Surrey RH6 7DQ (GB)**

(56) References cited:
**WO-A1-03/076702          WO-A1-2011/013075
WO-A2-2011/091074       DE-A1-102007 010 112
US-B1- 6 350 411           US-B1- 6 667 099**

EP 2 758 093 B1

**Description**

BACKGROUND

The main function of the lung is to exchange gasses between the ambient air and the blood. Within this framework $O_2$ is transferred from the environment to the blood while $CO_2$ is eliminated from the body.

In a normal resting human this process is associated with an $O_2$ input of about 200 - 250 $cm^3$/min and an output of about the same amount of $CO_2$. This exchange is made through a surface area of 50-100 $m^2$ of a 0.5 - 1 $\mu$m thick biological membrane separating the alveolar air from the pulmonary blood. The process is associated with the flow of similar volumes of blood and air - about 5 liter/min. At the given flow rate the blood is in "contact" with the membrane through which diffusion is taking place for a time period of 1/3 - 1/5 sec.

In natural systems such as the lung the gas exchange is achieved by diffusion taking place across a thin biological membrane separating two compartments: the gases in the lung alveoli and the gases contained in the blood of the lung capillaries. The gases in the alveolar compartment are maintained at a composition close to that of ambient air or gas by moving the air or gases in and out of the lungs by respiratory movements. The gas exchange is achieved by diffusion through the surface area of the exchange membrane that is extremely large - about 50 - 100 $m^2$. The driving force for diffusion of gases into and out of the blood is maintained by a very large blood flow through the lung capillaries.

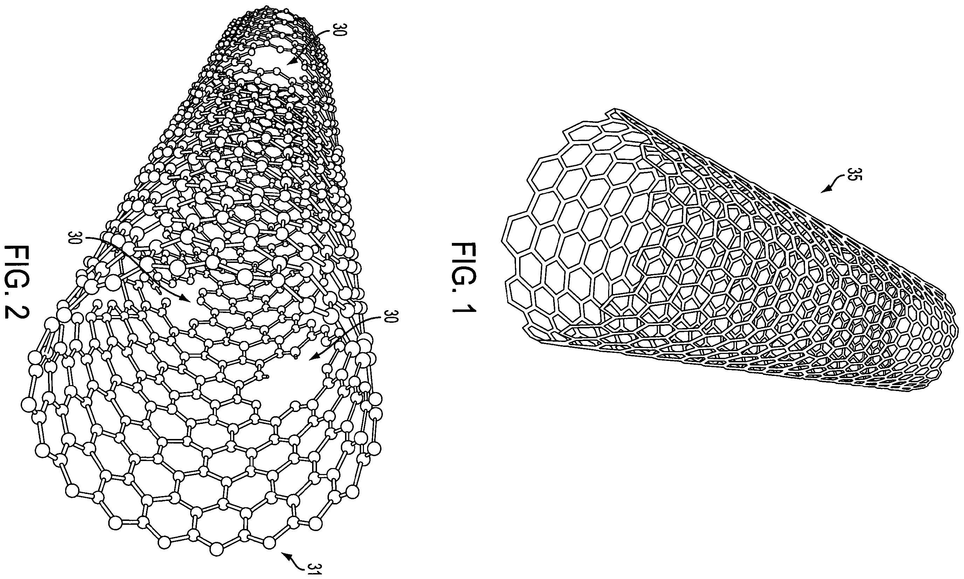

In the past decade carbon nanotubes became commercially available, and more recently nanotubes constructed from other materials (e.g., silicon) became available. These inert cylindrical structures have diameters of about 1-20 nm and walls constructed of a single layer of hexagonal carbon atom mesh 35, as seen in FIG. 1. Their length can reach values in the cm range. Nanotubes have been intensively studied in the past decade mainly as they conduct both heat and electricity and have exceptionally high mechanical strength.

Perfect carbon nanotubes are strong, flexible, and resilient, and are also impermeable to fluids including gases. In practice, however, the manufactured tubes have defects 30 in their walls 31 as illustrated in FIG. 2. Those defects act like pores that are gas permeable. These defects may be, for example, of crystallographic nature or in the form of atomic vacancies, etc. Another type of nanotube defect is the Stone Wales defect, which creates a pentagon and heptagon pair by rearrangement of the bonds. Moreover, such holes or pores can be purposely introduced e.g., by means of atomic force microscope systems.

WO-A-2011/013075 discloses a blood oxygenator device which has a substantially cylindrical structure containing a layer of capillaries made of a microporous membrane.

WO-A-2011/091074 discloses a silicon microporous membrane for oxygenating blood.

DE-A-102007010112 discloses a blood oxygenator which comprises a plurality of gas-permeable hollow fibers extending through a chamber.

WO-A-2003/076702 discloses a method for producing hollow fibers having an internal diameter in the nanonmeter to micrometer range.

US-A-6667099 discloses mesotubes and nanotubes having an internal diameter of 10 nm - 50 $\mu$m, and a method of producing the same.

SUMMARY OF THE INVENTION

One aspect of the invention is directed to a gas exchange unit for processing blood that includes red blood cells and plasma. The gas exchange unit includes a fluid-tight enclosure that has a front face with an input port for inputting the blood, a rear face with an output port for outputting the blood, an interior, and an exterior. The gas exchange unit also includes a bundle of nanotubes that run between the front face and the rear face, each of the nanotubes having a front end and a rear end, with a void in the bundle that extends from the input port on the front face to the output port on the rear face. The void is configured to form a flow channel that is large enough to permit the red blood cells to flow from the input port to the output port. The nanotubes in the bundle are spaced close enough to retain the red blood cells within the flow channel, yet far apart enough to permit the plasma to flow through spaces between adjoining nanotubes in the bundle. The nanotubes are arranged with respect to the front face and the rear face to permit $O_2$ molecules to diffuse into the nanotubes from the exterior of the enclosure and to permit $CO_2$ molecules to diffuse out of the nanotubes to the exterior of the enclosure. The nanotubes in the bundle have defects in their walls that permit $O_2$ molecules and $CO_2$ molecules to diffuse therethrough, and the defects are present in a sufficient number and total area so that the gas exchange unit can effectively deliver $O_2$ to the blood and carry away $CO_2$ from the blood.

In some preferred embodiments, the nanotubes are carbon nanotubes with a diameter between 5 and 20 nm, the front face and the rear face are between 0.3 and 3 cm apart, the nanotubes in the bundle, outside the void, are packed in at a density of at least 100 nanotubes per $\mu$m$^2$, and the flow channel has a cross section between 250 and 2500 $\mu$m$^2$.

In some preferred embodiments, the front face has a plurality of additional input ports for inputting the blood, the rear face has a plurality of additional output ports for outputting the blood, and the bundle of nanotubes has a plurality of additional voids that extend from the respective additional input ports to the respective additional output ports. The additional voids are configured to form additional flow channels that are large enough to permit the red blood cells to flow therethrough.

Another aspect of the invention is directed to a gas exchanger for processing blood that includes red blood cells and plasma. The gas exchanger includes at least eight gas exchange units, and each of the gas exchange units includes a fluid-tight enclosure with a front face with an input port for inputting the blood, a rear face with an output port for outputting the blood, an interior, and an exterior. Each of the gas exchange units also has a bundle of nanotubes that runs between the front face and the rear face, each of the nanotubes having a front end and a rear end, with a void in the bundle that extends from the input port on the front face to the output port on the rear face. The voids are configured to form flow channels that are large enough to permit the red blood cells to flow from the input port to the output port. The nanotubes in the bundles are spaced close enough to retain the red blood cells within the flow channels, yet far apart enough to permit the plasma to flow through spaces between adjoining nanotubes in the bundles. The nanotubes are arranged with respect to the front face and the rear face to permit $O_2$ molecules to diffuse into the nanotubes from the exterior of the enclosure and to permit $CO_2$ molecules to diffuse out of the nanotubes to the exterior of the enclosure. The nanotubes in the bundles have defects in their walls that permit $O_2$ molecules and $CO_2$ molecules to diffuse therethrough, and the defects are present in a sufficient number and total area so that the gas exchange unit can effectively deliver $O_2$ to the blood and carry away $CO_2$ from the blood. The gas exchanger also includes a plurality of gas flow channels arranged with respect to the gas exchange units to permit $O_2$ molecules to diffuse from the gas flow channels into the nanotubes in the gas exchange units and to permit $CO_2$ molecules to diffuse out of the nanotubes in the gas exchange units to the gas flow channels. The gas exchanger also includes at least four flow bridges, each of the flow bridges being configured to route blood from an output port of one of the plurality of gas exchange units to an input port of another one of the plurality of gas exchange units, and the flow bridges cross the gas flow channels.

In some preferred embodiments, in each of the gas exchange units, the nanotubes are carbon nanotubes with a diameter between 5 and 20 nm, the front face and the rear face are between 0.3 and 3 cm apart, the nanotubes in the bundle, outside the void, are packed in at a density of at least 100 nanotubes per $\mu m^2$, and the flow channel has a cross section between 250 and 2500 $\mu m^2$. In some preferred embodiments, the gas exchanger includes a pump configured to pump at least one of air, pure oxygen, and oxygenated air through the gas flow channels.

Also disclosed is a method of exchanging $O_2$ and $CO_2$ with blood that includes red blood cells and plasma. This method includes the steps of passing the blood through a void within a bundle of nanotubes. The void is configured to form a flow channel that is large enough to permit the red blood cells to flow from the input port to the output port, and the nanotubes in the bundle are spaced close enough to retain the red blood cells within the flow channel, yet far apart enough to permit the plasma to flow through spaces between adjoining nanotubes in the bundle. The nanotubes in the bundle have defects in their walls that permit $O_2$ molecules and $CO_2$ molecules to diffuse therethrough. This method also includes the steps of diffusing $O_2$ from a gas flow channel into the nanotubes, diffusing $O_2$ from the nanotubes into the blood through the defects, diffusing $CO_2$ from the blood into the nanotubes through the defects, and diffusing $CO_2$ from the nanotubes into the gas flow channel. The defects are present in a sufficient number and total area to effectively deliver $O_2$ to the blood and carry away $CO_2$.

In some preferred embodiments, the nanotubes are carbon nanotubes with a diameter between 5 and 20 nm, the nanotubes in the bundle, outside the void, are packed in at a density of at least 100 nanotubes per $\mu m^2$, and the flow channel has a cross section between 250 and 2500 $\mu m^2$.

Another aspect of the invention is directed to a gas exchanger that includes at least eight gas exchange units. Each of the gas exchange units includes a fluid-tight enclosure having a front face with an input port for inputting a liquid, a rear face with an output port for outputting the liquid, an interior, and an exterior. Each of the gas exchange units also includes a plurality of nanotubes that run between the front face and the rear face, each of the nanotubes having a front end and a rear end. The nanotubes are arranged with respect to the front face and the rear face to permit gas molecules to diffuse into the nanotubes from the exterior of the enclosure and to permit gas molecules to diffuse out of the nanotubes to the exterior of the enclosure, and the nanotubes have defects in their walls that permit gas molecules to diffuse therethrough. The defects are present in a sufficient number and total area so that the gas exchange unit can effectively deliver the gas to the liquid. The gas exchanger also includes a plurality of gas flow channels arranged with respect to the gas exchange units to permit gas molecules to diffuse from the gas flow channels into the nanotubes in the gas exchange units, and at least four flow bridges. Each of the flow bridges is configured to route liquid from an output port of one of the plurality of gas exchange units to an input port of another one of the plurality of gas exchange units, and the flow bridges cross the gas flow channels.

In some preferred embodiments, the nanotubes are carbon nanotubes with a diameter between 5 and 20 nm, the front face and the rear face are between 0.3 and 3 cm apart, and the nanotubes are packed in at a density of at least 100 nanotubes per $\mu m^2$. In some preferred embodiments, the gas exchanger also includes a pump configured to pump the gas through the gas flow channels.

BRIEF DESCRIPTION OF THE DRAWINGS

FIG. 1 depicts a conventional carbon nanotube.

FIG. 2 depicts a conventional carbon nanotube with pores in its wall.

FIG. 3A depicts a gas exchange system that mimics the function of human lung.

FIGS. 3B and 3C depict a preferred construction of a basic unit within the gas exchange system of FIG. 3A, from a side view and a perspective view, respectively.

FIG. 4A is a schematic representation of blood and gas flows through a basic unit of the gas exchange system.

FIG. 4B is a section view taken along section line B-B in FIG. 4A.

FIG. 5A is a perspective view of a blood flow channel in the middle of a bundle of nanotubes.

FIG. 5B is a schematic representation of a side view of the blood flow channel of FIG. 5A.

FIG. 5C depicts an embodiment in which 120 basic units are connected in series and parallel.

FIG. 6 is a more detailed view of a basic unit of the gas exchange system.

FIGS. 7A and 7B are schematic representations of external and internal gas exchange systems, respectively.

DESCRIPTION OF THE PREFERRED EMBODIMENTS

Some preferred embodiments of the invention relate to a gas exchanger ("GE") that is used to form an artificial lung for efficient gas exchange ($O_2$ and $CO_2$) between compartments such as human (or animal) blood and ambient air or some other gas. The GE system is a preferably based on matrix of parallel oriented Nanotubes. The ultra-small diameter of nanotubes, their gas permeability (when defects are present), their huge surface to volume ratio, together with their inert chemical nature are desirable characteristics in this context.

The system relies on diffusion through the walls of the nanotubes. In order for diffusion to occur, pores are created by introducing defects into the walls of the nanotubes using any suitable approach, as described above. Preferably, the defect concentration is at least 1 in $10^7$, and in some preferred embodiments, the defect concentration is on the order of 1 in $10^6$. A suitable pore size for the defects in the nanotube walls is on the order of 1 nm.

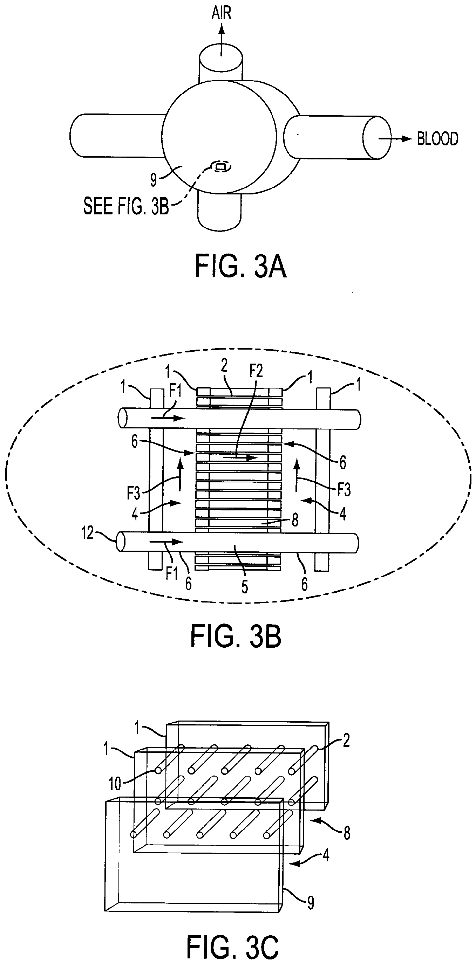

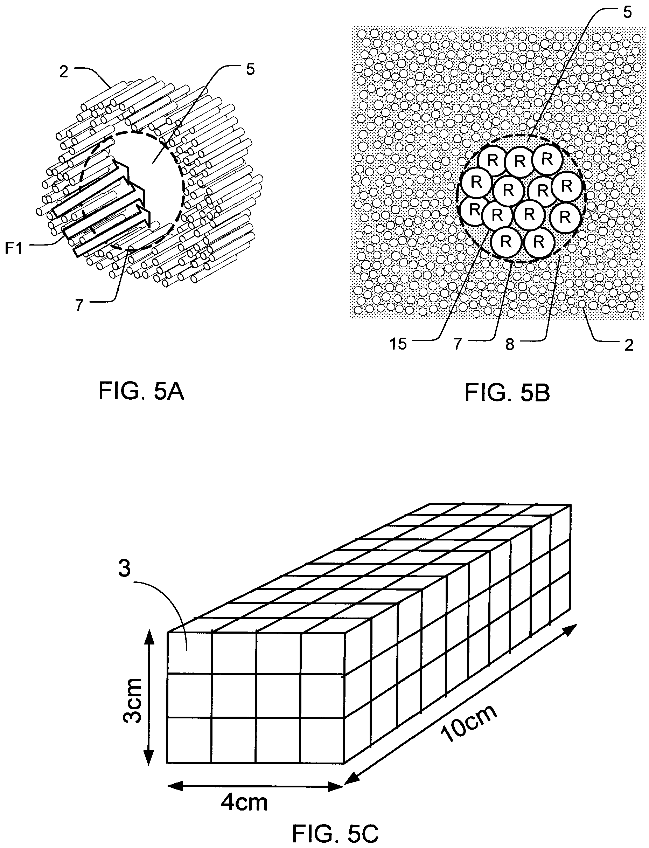

FIG. 3A depicts a gas exchange system that mimics the function of human lung. Gas exchange is preferably carried out in a number of basic units that are preferably connected both in parallel and in series, as shown in FIG. 5C, which depicts a 3x4x10 array of basic units 3. In the configuration illustrated in FIG. 5C, twelve basic units 3 are connected in parallel, and that block of twelve parallel basic units 3 is repeated ten times in series.

FIGS. 3B and 3C depict a preferred construction of one of these basic units, from a side view and a perspective view, respectively. An array of parallel nanotubes 2 spans the two parallel walls or supports 1, so that each basic unit containing multiple nanotubes 2 running in parallel from one support 1 to the other support 1. Methods for obtaining this configuration of parallel carbon nanotubes are described in J. Li, C. Papadopoulos, and J. M. Xua, Highly-ordered carbon nanotube arrays for electronics applications, Applied Physics Letters (1999); 75, 367-369.

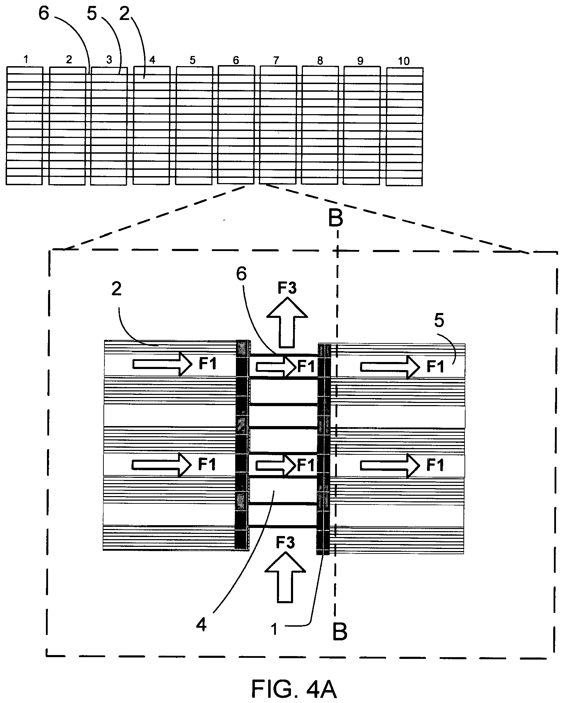

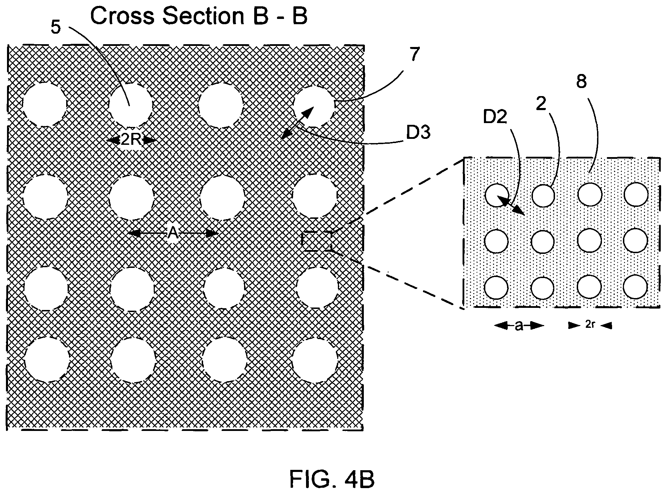

The nanotubes 2, having a diameter "2r" (shown in FIG. 4), and their corresponding openings at the supports 1, are preferably distributed in a matrix pattern, with center to center distances of "a", also shown in FIG. 4B. For example nanotubes with diameters of 2r = 10 nm may be arranged as a matrix such that their centers are a = 20 nm apart. In alternative embodiments, nanotubes with other diameters (e.g., between 5 and 20 nm) may be used, spaced apart at, for example, two or three times the diameter of the nanotubes.

Returning to FIG. 3B, the distance between the supports 1 in the basic unit is preferably 0.3 - 3 cm, depending on the nanotube length as they stretch across the gap between the supports 1. Nanotubes of such lengths are available. This nanotube length range was chosen that the gas diffusion along the nanotubes is sufficiently rapid to maintain the oxygen and $CO_2$ gas concentrations at the desired levels. In some preferred embodiments, nanotubes on the order of 1 cm are used. The nanotubes preferably penetrate the supports 1 through corresponding openings such that the ends of the nanotubes are open to, i.e., communicate with the space between the supports 1 of neighboring basic units. Those spaces are referred to herein as the gas flow channels 4, with the flow of gas F3 shown in FIG. 3B.

The matrix pattern of nanotubes is interrupted by voids, referred to herein as blood flow channels 5, best seen in FIGS. 4-5, which do not contain nanotubes. The blood flow channels 5 are preferably cylindrical, but can have other cross sections in alternative embodiments. A suitable cross section for the blood flow channels 5 is between 250 and 2500 $\mu$m$^2$. When cylindrical voids are used, a suitable diameter 2R is about 20 $\mu$. In some embodiments, the voids are preferably arranged as a matrix, e.g., with centers spaced about A=40 $\mu$ apart.

One preferred way for making a basic unit is to make the array parallel nanotubes that span from a front support 1 to a rear support 1, and then adding a fluid tight floor, ceiling, and side walls (not shown) to form a fluid-tight enclosure. A suitable size for this enclosure is a one cm cube. At this point, the entire space between the front and rear face is populated with nanotubes arranged in a matrix. Then, one or more blood flow channels 5 can be introduced by drilling from the front face through to the rear face to make voids in the matrix of nanotubes. A minimum of one blood flow channel 5 is present in each basic unit, but more than one blood flow channel 5 may also be made in each basic unit.

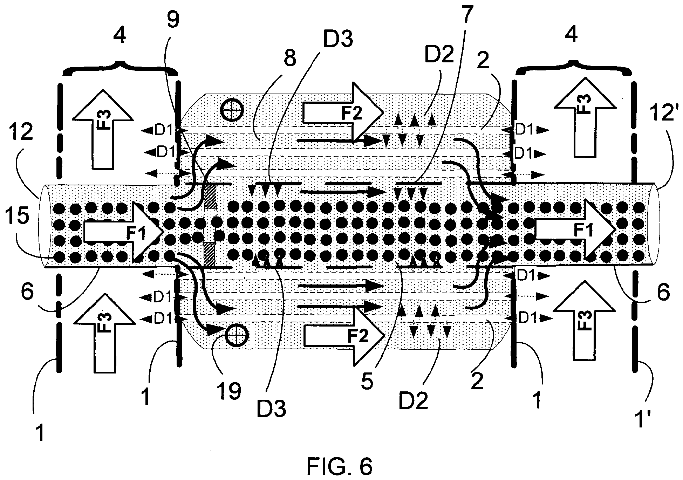

When made in this manner, each basic unit will have an input port in the front face, and an output port on the rear face. Blood enters the basic unit via the input port and travels through the blood flow channel 5 to the output port. Note that the borders of the blood flow channels 5 are virtual boundaries 7 formed by the nanotubes, as best seen in FIGS. 5A, 5B, and 6. As the nanotubes 2 that run in parallel with the blood flow channels 5 are packed much more closely (e.g., on 20 nm centers) than the diameter of the red blood cells 15 (which are on the order of 5 $\mu$m in diameter), the red blood cells 15 in the blood cannot exit the blood flow channels 5. However, the blood plasma (which is a liquid) can penetrate and flow in the space between the nanotubes 2, referred to herein as the plasma flow region 8. In other words, the nanotubes 2 in the bundle are spaced close enough to retain the red blood cells 15 within the blood flow channels 5, yet far apart enough to permit the plasma to flow through spaces between adjoining nanotubes in the bundle. A suitable spacing for the nanotubes to prevent the blood cells from leaving the blood flow channels 5 and allow for the appropriate gas exchanges is at least 100 nanotubes per $\mu$m$^2$.

Optionally, to help the wetting process, initially water, electrolyte, or plasma may be forced by pressure to occupy these spaces before the system is used to process the blood of a live patient.

In embodiments that have multiple basic units 3 connected in series, the blood must be transferred from a given basic unit 3 to the next unit in the series. This may be accomplished using flow bridges 6 to connect the blood flow channels 5 of two adjoining basic units (as best seen in FIGS. 3B, 4A, and 6) so as to form a continuous flow pathway. In particular, a flow bridge 6 will connect the output port from one basic unit to the input port of the next basic unit in the series (for series connections). Preferably, the basic units are aligned so that straight flow bridges 6 may be used. The flow bridges 6 may be connected to the input and output ports using a suitable adhesive.

In some preferred embodiments, the flow bridges 6 are thin walled tubes 12, 12' that are blood flow compatible (i.e. made so that the blood flowing through them is not damaged and does not coagulate). This compatibility can be achieved by the use of proper material, for example Teflon, or by coating the tubing by heparin or heparin like substances, etc. As to the contact of blond with the nanotubes at the virtual boundary 7, since the nanotubes are inert and biocompatible, and contact with them should be virtually frictionless, no biological damage to the blood cells is expected.

The system operates based on gas exchanges maintained by means of three separate channels of fluid flow (F1-F3) and three regions where gas diffusion processes occur (D1 - D3), as depicted schematically in FIG. 4A and in more detail in FIG. 6.

The three flows are best seen in FIG. 6: (1) blood flow F1 in the blood flow channels 5 and flow bridges 6; (2) plasma flow F2 around the nanotubes 2 in the plasma flow region 8; and (3) gas flow F3 in the gas flow channels 4.

The GE gas exchange is achieved primarily by gas diffusion, in three different processes: (1) along the hollow internal part of nanotubes 2 that are open at their two ends to relatively large gas flow channels 4, labeled D1; (2) across the very large surface area of the walls of the nanotubes 2 that contain the required gases (through the defects 30 shown in FIG. 2), labeled D2; and (3) across the virtual boundary 7 between the plasma in the plasma flow region 8 and the blood flow channel 5, labeled D3. The required gas composition within the nanotubes is maintained by diffusion from a flowing gas compartments into which the ends of the nanotube are open.

In this example the gas exchanger functions as follows: venous blood collected from the whole body is normally pumped by the right ventricle into the lungs. This blood, in whole or in part is diverted into the input of the GE where it flows through flow pathways F1 (for the red blood cell portion of the blood and a portion of the plasma) and F2 (for the rest of the plasma). In addition, either air, oxygen rich air, or oxygen is pumped into the gas flow channels 4, where it flows along path F3. Pumping may be implemented using any suitable conventional air pump that can maintain an adequate flow (e.g., 6L/min).

The first diffusion process (D1, shown in FIG. 6) is from the gas flow channels 4 (where the gas concentration is held constant by adequate gas flow) through the open ends of the nanotubes 2 that open into the gas flow channels 4. This process conforms to the well known diffusion process called effusion dealing with diffusion out of a relatively large container (here the gas flow channel 4) through a "pinhole" that corresponds to the open end of a nanotube. The mass transport M through the pinhole is given by:

$$M = 0.25 *P*(8m/\pi*R*T)^{0.5} *A*t$$

Where: P is pressure (atm), m is molecular weight, R is gas constant (ergldeg) $8.3*10^7$, T is temperature (Kelvin) 300°, A is area ($cm^2$), and t is time (sec).

Assuming the conditions of mass flow of 100% $O_2$ through a 1 $cm^2$ area M = 14.1 g/s or 0.44 Mol/s which gives 9694 $cm^3$/s, or 581,640 $cm^3$/min.

Further assuming an example in which the nanotube diameter is 10 nm; the distance between nanotube centers is 40 nm; the nanotube cross section area is $\pi(*(0.5*10^{-6})^2 = \pi* 0.25* 10^{-12} = 0.79*10^{-12}$ $cm^2$; and the number of nanotubes per $cm^2$ is $6.25*10^{10}$, the result is a total nanotube cross section of $4.91*10^{-2}$ $cm^2$ per each 1 $cm^2$ basic unit, and the $O_2$ flow capacity into the nanotubes in a 1 $cm^2$ basic unit is: $5.8*10^5 * 4.9*10^{-2}$, which comes to $2.8*10^4$ $cm^3$/min. This huge flow capacity (true also for 20% $O_2$) ensures that the $O_2$ concentration in the nanotubes will be kept practically constant despite the diffusion (D2) of gases across the nanotube surface. This conclusion is valid for relatively small length (about 1 mm - 1 cm) of the nanotubes 2 that are open to the gas flow channel 4 at their two ends.

The second diffusion process (D2) is in and out of the nanotubes 2 into the plasma that flows in the plasma flow region 8. In this case the $O_2$ transport can be estimated as follows. Assuming a nanotube diameter of 10 nm; nanotube surface area (for length of 1 cm): $\pi*10^{-6}$ $cm^2$, distance between nanotube centers of 40 nm; number of nanotubes per $cm^2$ of $6.25*10^{10}$, the total nanotube area per $cm^3$ comes to $1.96*10^5$ $cm^2$. Further assuming that the defect induced pores occupy 1/1,000,000 of nanotube surface area, the total surface available for diffusion will be 0.196 $cm^2$.

The $O_2$ diffusion coef. in air, D = 0.243 $cm^2$/s. The thickness of diffusion distance across a pore L =$10^{-7}$ cm (1 nm) when nanotube wall thickness is 0.3 nm. So $\Delta C = 1.8*10^{-3}$ /22000= $8.2*10^{-8}$ $MolO_2$/cc (see below)

The $O_2$ transport per I $cm^3$ Unit is therefore dn/dt = D*A*dC/dX = $0.243*0.196* 8.2*10^{-8}$ /$10^{-7}$ = $3.9*10^{-2}$ Mol $O_2$/s, and multiplying by 60, 2.34 $MolO_2$/min.

The transport in $cm^3$/s is: $0.243*0.196* 1.8*10^{-3}$ /$10^{-7}$ = 860 $cm^3$/s. Multiplying by 60, this comes to 51,600 $cm^3$/min for a single unit, which is well in excess of any need for a human.

The above results are a function of the nanotube density in a linear fashion such that if for example the distance between nanotube centers is 20 nm rather than 40 nm, the nanotubes would be packed in 4 times more densely, in which case the flow would be four times as high, i.e., 3440 $cm^3$/s.

The Third diffusion process (D3) is of the relevant gases, $O_2$ and $CO_2$, in the plasma (located in the plasma flow region 8) and blood flow channel 5 across the virtual boundary 7.

The exchange capacity of this process can be calculated for $O_2$ as follows: The relevant $O_2$ diffusion occurs between two bodies of essentially water (electrolyte) where the solubility ratio (water to water), S = 1. Let us consider the case where the $O_2$ content of the flowing blood in the blood flow channel 5 is same as typical venous blood while the $O_2$ content in the plasma (located in the plasma flow region 8) that exchanges gases in the nanotubes is that of arterial blood.

Assuming the following conditions: Venous partial pressure of oxygen ($PvO_2$) = 40 mm Hg; Arterial partial pressure of oxygen ($PaO_2$) = 100 mm Hg; and $O_2$ content = .003 ml $O_2$/dl/mm Hg, we obtain

$O_2$ content (100 mmHg) arterial like plasma = 0.3 $cm^3O_2$/dl = $3*10^{-3}$ ml $O_2$ /cc; and
$O_2$ content (40 mmHg) venous like plasma = 0.12 $mlO_2$/dl = $1.2*10^{-3}$ ml $O_2$ /cc, so

$$\Delta C = 3*10^{-3} - 1.2*10^{-3} = 1.8*10^{-3} \ cm^3O_2/cc.$$

Converting to Moles, we obtain

$$\Delta C = 1.8*10^{-3} /22000 = 8.2*10^{-8} \ Mol \ O_2/cc.$$

The thickness W of the virtual membrane across which the concentration gradient is maintained is: $10^{-4}$ cm (1μ); the diffusion coefficient of $O_2$ in water D = $3*10^{-5}$ $cm^2$/s; and the permeability coefficient of $O_2$: P = D/W = $3*10^{-5}$ cm/s /$10^{-4}$ = 0.3.

$O_2$ diffusion across an area of 1 $cm^2$ is given by:

$$dn/dt = P*S*A*\Delta C = 0.3 *1*1*1.8*10\text{-}3 = 0.6*10\text{-}3 \ cm^3 \ O_2 \ /s$$

For a system having 1 cm long blood flow channels 5 with diameter of $2*10^{-3}$ (20 $\mu$), the flow channel circumference will be $2\pi * 10^{-3}$ cm. If the distance between flow channel centers is $3*10^{-3}$ (30 $\mu$), the number of channels/cm$^2$ is $10^5$. The total flow channel surface area A will then be $2\pi *10^{-3}*10^5$ = 628 cm$^2$.

The Total $O_2$ Transport across the flow channels' surface in a single 1 cm$^3$ basic unit will be T= $0.6*10^{-3}*628$ = 0.38 cm$^3$ $O_2$ /s or 0.38*60 = 22.8 cm$^3$/min, so in a GE consisting of 120 basic units arranged as illustrated in FIGS. 4 & 5, the total oxygen transport will be 22.8*120 = 2736 cm$^3$/min. This is well beyond the $O_2$ requirement of a resting human, which is 250 cm$^3$/min.

Note that all the diffusion capacities calculated above were for $O_2$, at atmospheric pressure, flowing in the gas flow channel 4. These values can be multiplied by a factor of about 5 by replacing the air that contains about 20% $O_2$ with 100% oxygen. Furthermore, the gas pressure can be elevated above the atmospheric pressure together with the pressure of the other relevant elements in the system to further improve the gas exchange.

As for the efficacy of the Gas Exchanger with regards to $CO_2$, the water diffusion coefficients of $CO_2$ and $O_2$ are similar while the solubility of $CO_2$ is about 24 times higher than that of $O_2$. As the $O_2$ and $CO_2$ concentration difference between oxygenated and reduced blood are similar, the diffusion rate of $CO_2$ is about 20 times that of $O_2$. Thus, the $CO_2$ transport in all the above processes is expected to be superior to that or $O_2$.

As mentioned above there are three fluid flows in the GE, best seen in FIG. 6: (1) blood flow F1 in the blood flow channels 5 and flow bridges 6; (2) plasma flow F2 around the nanotubes (in the plasma flow region 8); and (3) gas flow F3 in the gas flow channels 4. All flows may be measured by appropriate flow meters and controlled by changes in pressure driving force as well as resistance to flow. Analog or digital controllers, that have access to the measured flow rates, the relevant physiological parameters, the pressure generators, and the flow resistance controllers, may be used to maintain the desired flows.

The blood flow through the GE (which includes the flow F1 of the red blood cells and the flow F2 of the plasma, both shown in FIG. 6) may be obtained from the pulmonary artery or any other vessel carrying a sufficient amount of venous blood- The flows F1 and F2 can be maintained by the natural pressure generated by the right ventricle or other parts of the circulation. Alternatively, flows F1 and F2 can be driven by an external or implanted pump designed to generate blood flow for long periods of time. Pumps similar to the Jarvik-7 heart, the SynCardia Systems Artificial Heart (formerly known as the Cardio West TAH), and the AbioCor Replacement Heart by AbioMed and the like are suitable for this purpose. The blood exiting the GE can then be introduced back into the patient via a pulmonary vein or veins, or any other appropriate blood vessel.

The flow rate is preferably adjustable to match the needs of the person, organism etc. this adjustment may be dynamic according to the changing need, for example, by increasing the flow rate during exercise. The adjustment may be controlled by sensors of a relevant physiological parameter such as the partial pressure of $O_2$ and/or $CO_2$ in the blood, oxygen Hb saturation (oximetry), pH, etc. (e.g., by increasing flow when a drop in $O_2$ is detected).

To supply the $O_2$ (or other gas) needs, which amount to approximately 250 cm$^3$/min for a resting adult man, a flow of about 5-6 L/min oxygenated blood is required. An additional factor that is preferably taken into consideration is the time the flowing blood is exposed to the gas diffusion process, the dwell time. In the normal resting human lung this duration is about 1/3 - 1/5 of a see while the flow velocity is usually under 100 cm/s.

In the described GE the total cross section of the blood flow channels 5 is 3.6 cm$^2$ while its length is 10 cm (assuming there are ten 1 cm basic units connected in series). Assuming flow F1 of 6L/min = 100 cm$^3$/s, we have a dwell time of 1/10s. As the diffusion capacity of GE is at least one order of magnitude in excess of the need, this somewhat shorter time should not hamper adequate gas exchange.

The second flow F2 is that of the plasma around the nanotubes (in the plasma flow region 8). The blood that flows into the GE consists mainly of red blood cells ("RBCs") and plasma. While the plasma can flow in both the blood flow channel 5 and the areas around the nanotubes, the spaces between the nanotubes (about 20 -50 nm) are too small to accommodate the RBCs which have diameters of 5-8$\mu$. Thus, there is separation of the blood into two: plasma and RBCs with a little amount of plasma around the (high hematocrit). Both flows are slowed down, the plasma by the presence of the numerous nanotubes along their path and the RBCs by the high viscosity of the concentrated blood.

The GE may be equipped with a pump (e.g., a peristaltic pump that only partly occludes the tubing while propelling the fluid) to overcome this slowing and maintain adequate flows. Special attention is preferably given to maintain the balance between the two flows such that at the GE exit the blood is properly reconstituted. This may be achieved by passive flow control devices like a flow restrictor, or by an active control system that individually controls the pressure gradient and/or the resistance to flow in the plasma pathway F2 and/or the RBC pathway F1.

For example, as seen in FIG. 6, the resistance to flow F1 maybe increased using a flow resistance controller 9, which may be implemented using a mechanical device like a louver, shutter, or another orifice diameter control mechanism.

When the blood enters the basic unit, some of the plasma is deviated from the blood flow channel 5 to the plasma flow region 8. In some embodiments, about 50% of the total blood plasma should be deviated, and the flow resistance controller 9 is preferably configured to assist this deviation. Alternatively or in addition, the separation process may be aided by flow enhancer 19 that increases the pressure gradient and flow rate in the plasma flow region 8. Examples of suitable mechanisms for the flow enhancer 19 include peristaltic pumps.

As indicated by the arrows on the right side of FIG. 6, the plasma flow F2 rejoins the RBC flow F1 towards the rear part of the basic unit 3. Note that in alternative embodiments, the need for the blood separation can be reduced or eliminated by increasing the distances between the nanotubes to reduce the flow restriction, and increasing the size of the device to compensate for the loss in surface area.

The gas that flows (F3 in FIGS. 3B, 4A, and 6) in the gas flow channels 4 is preferably oxygen, but air or other gas mixtures can be used. The gas flow inlet tubes can be connected to any gas source where pressure and composition is regulated. The corresponding outlet may lead to a collecting compartment or the gases may be discharged to the environment. The gas flow may be maintained by an appropriate pressure gradient that originated from a compressed gas container or a pumping system. The flow is preferably controlled using any of a variety of conventional approaches. The flow rate is preferably such that the gas concentration at the origin of the nanotubes is practically constant. As an example, the amount of gas diffusing away from the nanotubes in the case of an artificial lung is about 250 $cm^3$/min in a resting subject and may be more than 10 times this value during exercise. Thus, flows of about 10 - 100 L/min would suffice in most cases. The average gas pressure in the gas flow channels 4 is preferably similar to the atmospheric pressure. However, in alternative embodiments it may be different, e.g. elevated so as to generate higher transport by diffusion, etc. In such cases the pressure of the blood in the blood flow channel 5 may have to be elevated to corresponding levels.

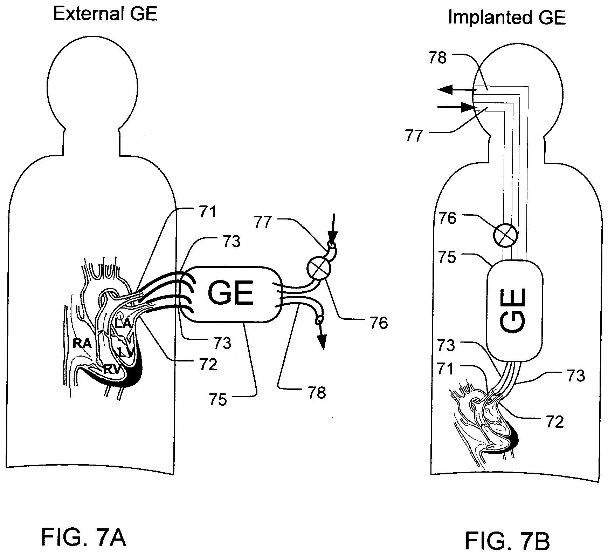

FIGS. 7A and 7B are schematic representations of how the GF. can be attached to a patient, for external and internal GEs respectively. In either case, the blood enters the GE 75 via tubing 73 from the pulmonary artery 71 and the blood is returned to the pulmonary vein from the GE via tubing 73. Air or oxygen is pumped into the GE 75 via the gas input tube 77 by pump 76, and the exhaust leaves via the exhaust tube 78. In these embodiments, the pulmonary artery can be used as the blood source to the GE and the one of the pulmonary veins (before they reach the left atrium) can be used as a drain for the oxygenated blood. In such a case the GE 75 replaces the lung. However, in alternative embodiments, the GE 75 may be connected in parallel to a failing respiratory system. The GE 75 can also be introduced into the peripheral circulation to enhance the blood gas exchanges. In such a case, for example, the GE can be connected in parallel to the femoral vein or introduced between distal and proximal incisions into the femoral vein. In such case the flow rates will be smaller than those described above. Note that the blood that flowed through the GE may be returned to circulation through the pulmonary vein as shown in FIGS. 7A and 7B, or to a different blood vessel.

The invention is described above in the context of delivering $O_2$ to blood and removing $CO_2$ from blood. But the invention is not limited to that application, and can be used to deliver other gases to blood. For example, it may be used in connection with a body part that has a dedicated circulation (such as a leg, brain, kidney), to deliver any desired gas to that body part. This can be used to deliver a chemical such as an anesthetic or therapeutic gas intended to act locally. In such a case the gas will be inputted into the artery and outputted (eliminated) via the vein, etc.

Note that in other types of GEs, fluids other than blood may be utilized. In cases where the fluid is homogeneous, the separation system (i.e., the blood flow channels 5 within the bundle of nanotubes) would not be needed, and the entire basic unit can be filled with the nanotubes. The invention is also not limited to medical uses, and can be used to exchange gases in other types of fluid flow systems, including industrial applications.

While the present invention has been disclosed with reference to certain embodiments, numerous modifications, alterations, and changes to the described embodiments are possible without departing from the scope of the present invention, as defined in the appended claims. Accordingly, it is intended that the present invention not be limited to the described embodiments, but that it has the full scope defined by the language of the following claims.

## Claims

1. A gas exchange unit for processing blood that includes red blood cells and plasma, the gas exchange unit being **characterized by**:

   a fluid-tight enclosure having a front face with an input port for inputting the blood, and a rear face with an output port for outputting the blood, the fluid-tight enclosure having an interior and an exterior; and
   a bundle of nanotubes (2) that run between the front face and the rear face, each of the nanotubes (2) having a front end and a rear end, with a void (5) in the bundle that extends from the input port on the front face to the output port on the rear face, the void (5) being configured to form a flow channel that is large enough to permit the red blood cells to flow from the input port to the output port,

   wherein the nanotubes (2) in the bundle are spaced close enough to retain the red blood cells within the flow channel, yet far apart enough to permit the plasma to flow through spaces between adjoining nanotubes (2) in the bundle,

wherein the nanotubes (2) are arranged with respect to the front face and the rear face to permit $O_2$ molecules to diffuse into the nanotubes (2) from the exterior of the enclosure and to permit $CO_2$ molecules to diffuse out of the nanotubes (2) to the exterior of the enclosure, and

wherein the nanotubes (2) in the bundle have defects in their walls that permit $O_2$ molecules and $CO_2$ molecules to diffuse therethrough, and the defects are present in a sufficient number and total area so that the gas exchange unit can effectively deliver $O_2$ to the blood and carry away $CO_2$ from the blood.

2. The gas exchange unit of claim 1, wherein the nanotubes (2) are carbon nanotubes.

3. The gas exchange unit of claim 1, wherein the front face and the rear face are between 0.3 and 3 cm apart.

4. The gas exchange unit of claim 1, wherein the nanotubes (2) have a diameter between 5 and 20 nm.

5. The gas exchange unit of claim 1, wherein the nanotubes (2) in the bundle, outside the void (5), are packed in at a density of at least 100 nanotubes per $\mu m^2$.

6. The gas exchange unit of claim 1, wherein the flow channel has a cross section between 250 and 2500 $\mu m^2$.

7. The gas exchange unit of claim 1, wherein the font face has a plurality of additional input ports for inputting the blood, the rear face has a plurality of additional output ports for outputting the blood, and the bundle of nanotubes (2) has a plurality of additional voids (5) that extend from the respective additional input ports to the respective additional output ports, the additional voids (5) being configured to form additional flow channels that are large enough to permit the red blood cells to flow therethrough.

8. The gas exchange unit of claim 1 or 8, wherein the nanotubes (2) are carbon nanotubes with a diameter between 5 and 20 nm, the front face and the rear face are between 0.3 and 3 cm apart, the nanotubes (2) in the bundle, outside the void (5), are packed in at a density of at least 100 nanotubes per $\mu m^2$, and the flow channel has a cross section between 250 and 2500 $\mu m^2$.

9. A gas exchanger for processing blood that includes red blood cells and plasma, the gas exchanger being **characterized by**:

   at least eight gas exchange units (3), wherein each of the gas exchange units (3) includes a fluid-tight enclosure having a front face with an input port for inputting the blood, a rear face with an output port for outputting the blood, the fluid-tight enclosure having an interior and an exterior, and
   a bundle of nanotubes (2) that run between the front face and the rear face, each of the nanotubes (2) having a front end and a rear end, with a void (5) in the bundle that extends from the input port on the front face to the output port on the rear face, the void (5) being configured to form a flow channel that is large enough to permit the red blood cells to flow from the input port to the output port,

   wherein the nanotubes (2) in the bundle are spaced close enough to retain the red blood cells within the flow channel, yet far apart enough to permit the plasma to flow through spaces between adjoining nanotubes (2) in the bundle, wherein the nanotubes (2) are arranged with respect to the front face and the rear face to permit $O_2$ molecules to diffuse into the nanotubes (2) from the exterior of the enclosure and to permit $CO_2$ molecules to diffuse out of the nanotubes (2) to the exterior of the enclosure, and
   wherein the nanotubes (2) in the bundle have defects in their walls that permit $O_2$ molecules and $CO_2$ molecules to diffuse therethrough, and the defects are present in a sufficient number and total area so that the gas exchange unit (3) can effectively deliver $O_2$ to the blood and carry away $CO_2$ from the blood;
   a plurality of gas flow channels (4) arranged with respect to the gas exchange units (3) to permit $O_2$ molecules to diffuse from the gas flow channels (4) into the nanotubes (2) in the gas exchange units (3) and to permit $CO_2$ molecules to diffuse out of the nanotubes (2) in the gas exchange units (3) to the gas flow channels (4); and
   at least four flow bridges (6), each of the flow bridges (6) being configured to route blood from an output port of one of the plurality of gas exchange units (3) to an input port of another one of the plurality of gas exchange units (3), wherein the flow bridges (6) cross the gas flow channels (4).

10. The gas exchanger of claim 9, wherein, in each of the gas exchange units (3), the font face has a plurality of additional input ports for inputting the blood, the rear face has a plurality of additional output ports for outputting the blood, and the bundle of nanotubes (2) has a plurality of additional voids (5) that extend from the respective additional input ports to the respective additional output ports, the additional voids (5) being configured to form additional flow channels that are large enough to permit the red blood cells to flow therethrough, and further comprising:

> a plurality of additional flow bridges (6) that connect respective additional output ports to respective additional input ports.

**11.** The gas exchanger of claim 9 or 10, wherein, in each of the gas exchange units (3), the nanotubes (2) are carbon nanotubes with a diameter between 5 and 20 nm, the front face and the rear face are between 0.3 and 3 cm apart, the nanotubes (2) in the bundle, outside the void (5), are packed in at a density of at least 100 nanotubes per $\mu m^2$, and the flow channel has a cross section between 250 and 2500 $\mu m^2$.

**12.** The gas exchanger of claim 9, further comprising:

> a first pump (76) configured to pump at least one of air, pure oxygen, and oxygenated air through the gas flow channels (4), optionally further comprising: a second pump configured to pump the blood through the gas exchange units (3).

**13.** A gas exchanger, **characterized by**:

> at least eight gas exchange units (3), wherein each of the gas exchange units (3) includes a fluid-tight enclosure having a front face with an input port for inputting a liquid, and a rear face with an output port for outputting the liquid, the fluid-tight enclosure having an interior and an exterior, and
> a plurality of nanotubes (2) that run between the front face and the rear face, each of the nanotubes (2) having a front end and a rear end,

> wherein the nanotubes (2) are arranged with respect to the front face and the rear face to permit gas molecules to diffuse into the nanotubes (2) from the exterior of the enclosure and to permit gas molecules to diffuse out of the nanotubes (2) to the exterior of the enclosure, and
> wherein the nanotubes (2) have defects in their walls that permit gas molecules to diffuse therethrough, and the defects are present in a sufficient number and total area so that the gas exchange unit (3) can effectively deliver the gas to the liquid; a plurality of gas flow channels (4) arranged with respect to the gas exchange units (3) to permit gas molecules to diffuse from the gas flow channels (4) into the nanotubes (2) in the gas exchange units (3); and
> at least four flow bridges (6), each of the flow bridges (6) being configured to route liquid from an output port of one of the plurality of gas exchange units (3) to an input port of another one of the plurality of gas exchange units (3), wherein the flow bridges (6) cross the gas flow channels (4).

**14.** The gas exchanger of claim 13, wherein, in each of the gas exchange units (3), the nanotubes (2) are carbon nanotubes with a diameter between 5 and 20 nm, the front face and the rear face are between 0.3 and 3 cm apart, and the nanotubes (2) are packed in at a density of at least 100 nanotubes per $\mu m^2$.

**15.** The gas exchanger of claim 13, further comprising:

> a first pump (76) configured to pump the gas through the gas flow channels (4),
> optionally further comprising:

> > a second pump configured to pump the liquid through the gas exchange units (3).

**Patentansprüche**

**1.** Gasaustauscheinheit zur Verarbeitung von Blut, das rote Blutkörperchen und Plasma umfasst, worin die Gasaustauscheinheit **gekennzeichnet ist durch**:

> eine Fluid-dichte Umhüllung mit einer Vorderseite, die eine Einlaßöffnung zur Zuführung des Bluts aufweist, und einer Rückseite, die eine Auslaßöffnung zur Ausgabe des Bluts aufweist, worin die Fluid-dichte Umhüllung ein Inneres und ein Äußeres aufweist; und
> ein Bündel Nanoröhrchen (2), das zwischen der Vorderseite und der Rückseite verläuft, worin jedes der Nanoröhrchen (2) ein vorderes Ende und ein hinteres Ende aufweist, mit einem Leerraum (5) in dem Bündel, der sich von der Einlaßöffnung auf der Vorderseite zu der Auslaßöffnung auf der Rückseite erstreckt, worin der Leerraum (5) ausgestaltet ist, einen Strömungskanal auszubilden, der groß genug ist, so dass die Blutkörperchen von der Einlaßöffnung zu der Auslaßöffnung strömen können,

worin die Nanoröhrchen (2) in dem Bündel ausreichend beanstandet sind, um die roten Blutkörperchen in dem Strömungskanal zu halten, jedoch ausreichend weit beabstandet sind, so dass das Plasma **durch** den Raum zwischen benachbarten Nanoröhrchen (2) in dem Bündel strömen können,
worin die Nanoröhrchen (2) bezüglich der Vorderseite und der Rückseite angeordnet sind, so dass $O_2$-Moleküle von dem Äußeren der Umhüllung in die Nanoröhrchen (2) hineindiffundieren können und $CO_2$-Moleküle aus den Nanoröhrchen (2) zu dem Äußeren der Umhüllung hinausdiffundieren können, und
worin die Nanoröhrchen (2) in dem Bündel in deren Wänden Fehler aufweisen, so dass $O_2$-Moleküle und $CO_2$-Moleküle hindurch diffundieren können, wobei die Fehler in einer ausreichenden Anzahl und Gesamtbereich vorhanden sind, so dass die Gasaustauscheinheit wirksam $O_2$ ins Blut und $CO_2$ aus dem Blut liefern kann.

2. Gasaustauscheinheit nach Anspruch 1, worin die Nanoröhrchen (2) Carbon-Nanoröhrchen sind.

3. Gasaustauscheinheit nach Anspruch 1, worin die Vorderseite und die Rückseite zwischen 0,3 und 3 cm entfernt sind.

4. Gasaustauscheinheit nach Anspruch 1, worin die Nanoröhrchen (2) einen Durchmesser von zwischen 5 und 20 nm aufweisen.

5. Gasaustauscheinheit nach Anspruch 1, worin die Nanoröhrchen (2) in dem Bündel, ausserhalb des Leerraums (5), in einer Dichte von mindestens 100 Nanoröhrchen per $\mu m^2$ gepackt sind.

6. Gasaustauscheinheit nach Anspruch 1, worin der Strömungskanal einen Querschnitt von zwischen 250 und 2500 $\mu m^2$ aufweist.

7. Gasaustauscheinheit nach Anspruch 1, worin die Vorderseite mehrere zusätzliche Einlaßöffnungen zur Zuführung von Blut aufweist, worin die Rückseite mehrere zusätzliche Auslaßöffnungen zur Ausgabe des Bluts aufweist, und worin das Bündel Nanoröhrchen (2) mehrere zusätzliche Leerräume (5) aufweist, die sich von der jeweiligen zusätzlichen Einlaßöffnungen zu den jeweiligen zusätzlichen Auslaßöffnungen erstrecken, worin die zusätzlichen Leerräume (5) ausgestaltet sind, zusätzliche Strömungskanäle auszubilden, die ausreichend groß sind, so dass rote Blutkörperchen durchfließen können.

8. Gasaustauscheinheit nach Anspruch 1 oder 8, worin die Nanoröhrchen (2) Carbon-Nanoröhrchen mit einem Durchmesser zwischen 5 und 20 nm sind, worin die Vorderseite und die Rückseite zwischen 0,3 und 3 cm entfernt sind, worin die Nanoröhrchen (2) in dem Bündel, außerhalb des Leerraums (5), in einer Dichte von mindestens 100 Nanoröhrchen per $\mu m^2$ gepackt sind, und der Strömungskanal einen Querschnitt von zwischen 250 und 2500 $\mu m^2$ aufweist.

9. Gasaustauscher zur Verarbeitung von Blut, welches rote Blutkörperchen und Plasma umfasst, worin der Gasaustauscher **gekennzeichnet ist durch**:

mindestens acht Gasaustauscheinheiten (3), worin jede der Gasaustauscheinheiten (3) eine Fluid-dichte Umhüllung mit einer Vorderseite mit einer Einlaßöffnung zur Zuführung des Bluts, einer Rückseite mit einer Auslaßöffnung zur Ausgabe des Bluts aufweist, worin die Fluid-dichte Umhüllung ein Inneres und ein Äußeres aufweist, und
ein Bündel Nanoröhrchen (2), die zwischen der Vorderseite und der Rückseite verläuft, worin jedes der Nanoröhrchen (2) ein vorderes Ende und ein hinteres Ende aufweist, worin sich ein Leerraum (5) in dem Bündel von der Einlaßöffnung auf der Vorderseite zu der Auslaßöffnung auf der Rückseite erstreckt,

worin der Leerraum (5) ausgestaltet ist, einen Strömungskanal auszubilden, der ausreichend groß ist, so dass die roten Blutkörperchen von der Einlaßöffnung zu der Auslaßöffnung strömen können,
worin die Nanoröhrchen (2) in dem Bündel ausreichend nahe beabstandet sind, so dass die roten Blutkörperchen in dem Strömungskanal bleiben, jedoch ausreichend weit beabstandet, so dass das Plasma **durch** die Räume zwischen benachbarten Nanoröhrchen (2) in dem Bündel strömen kann,
worin die Nanoröhrchen (2) bezüglich der Vorderseite und der Rückseite angeordnet sind, so dass $O_2$-Moleküle von dem Äußeren der Umhüllung in die Nanoröhrchen (2) hineindiffundieren können und $CO_2$-Moleküle aus den

Nanoröhrchen (2) zu dem Äußeren der Umhüllung hinausdiffundieren können, und

worin die Nanoröhrchen (2) in dem Bündel Fehler in den Wänden aufweisen, so dass $O_2$-Moleküle und $CO_2$-Moleküle hindurchdiffundieren können, und worin die Fehler in einer ausreichenden Anzahl und einem Gesamtbereich vorhanden sind, so dass die Gasaustauscheinheit (3) wirksam $O_2$ zu dem Blut und $CO_2$ weg von dem Blut tragen kann; mehrere Gas-Strömungskanäle (4), die bezüglich der Gasaustauscheinheiten (3) angeordnet sind, so dass $O_2$-Moleküle von den Gas-Strömungskanälen (4) in die Nanoröhrchen (2) in den Gasaustauscheinheiten (3) strömen können und so dass $CO_2$- Moleküle aus den Nanoröhrchen (2) in den Gasaustauscheinheiten (3) zu den Gas-Strömungskanälen (4) diffundieren können; und

mindestens vier Strömungsbrücken (6), worin jede der Strömungsbrücken (6) ausgestaltet ist, Blut von einer Auslaßöffnung einer der mehreren Gasaustauscheinheiten (3) zu einer Einlaßöffnung einer anderen der mehreren Gasaustauscheinheiten (3) zu leiten, worin die Strömungsbrücken (6) die Strömungskanäle (4) kreuzen.

10. Gasaustauscher nach Anspruch 9, worin, in jeder der Gasaustauscheinheiten (3), die Vorderseite mehrere zusätzliche Einlaßöffnungen zur Zuführung von Blut aufweist, und die Rückseite mehrere zusätzliche Auslaßöffnungen zur Ausgabe des Bluts aufweist, und worin das Bündel Nanoröhrchen (2) mehrere zusätzliche Leerräume (5) aufweist, die sich von den jeweiligen zusätzlichen Einlaßöffnungen zu den jeweiligen zusätzlichen Auslaßöffnungen erstrecken, worin die zusätzlichen Leerräume ausgestaltet sind (5), zusätzliche Strömungskanäle auszubilden, die ausreichend groß sind, so dass rote Blutkörperchen durchströmen können, und weiter umfassend:

mehrere zusätzliche Strömungsbrücken (6), die die jeweiligen zusätzlichen Ausgabeöffhungen mit den jeweiligen zusätzlichen Einlaßöffnungem verbinden.

11. Gasaustauscher nach Anspruch 9 oder 10, worin in jeder der Gasaustauscheinheiten (3), die Nanoröhrchen (2) Carbon-Nanoröhrchen mit einem Durchmesser zwischen 5 und 20 nm sind, worin die die Vorderseite und die Rückseite zwischen 0.3 und 3 cm entfernt sind, und worin die Nanoröhrchen (2) in dem Bündel, außerhalb des Leerraums void (5), in einer Dichte von mindestens 100 Nanoröhrchen per $\mu m^2$ gepackt sind, und worin der Strömungskanal einen Querschnitt zwischen 250 und 2500 $\mu m^2$ aufweist.

12. Gasaustauscher nach Anspruch 9, welcher weiter umfasst:

eine erste Pumpe (76), die ausgestaltet ist mindestens eines von Luft, reinem Sauerstoff und mit Sauerstoff versetzter Luft durch die Gas-Strömungskanäle (4) zu pumpen, wahlweise weiter umfassend:

eine zweite Pumpe, die ausgestaltet ist, das Blut durch die Gasaustauscheinheiten (3) zu pumpen.

13. Gasaustauscher, **gekennzeichnet durch**:

mindestens acht Gasaustauscheinheiten (3), worin jeder der Gasaustauscheinheiten (3) eine Fluid-dichte Umhüllung mit einer Vorderseite mit einer Einlaßöffnung zur Zuführung einer Flüssigkeit, und einer Rückseite mit einer Auslaßöffnung zur Ausgabe der Flüssigkeit umfasst, worin die Fluid-dichte Umhüllung ein Inneres und ein Äußeres aufweist, und

mehrere Nanoröhrchen (2), die zwischen der Vorderseite und der Rückseite verlaufen, worin jedes der Nanoröhrchen (2) ein vorderes Ende aufweist und ein hinteres Ende, worin die Nanoröhrchen (2) bezüglich der Vorderseite und der Rückseite angeordnet sind, so dass Gasmoleküle von dem Äußeren der Umhüllung in die Nanoröhrchen (2) diffundieren können und Gas-Moleküle aus den Nanoröhrchen (2) zu dem Äußeren der Umhüllung diffundieren können, und worin die Nanoröhrchen (2) in deren Wänden Fehler aufweisen, so dass Gas-Moleküle hindurchdiffundieren können, und worin die Fehler in einer ausreichenden Anzahl und einem Gesamtbereich vorhanden sind, so dass die Gasaustauscheinheit (3) das Gas wirksam zu der Flüssigkeit liefern kann;

mehrere Gas-Strömungskanäle (4), die bezüglich der Gasaustauscheinheiten (3) angeordnet sind, so das Gas-Moleküle von den Gas-Strömungskanälen (4) in die Nanoröhrchen (2) in den Gasaustauscheinheiten (3) diffundieren können; und

mindestens vier Strömungsbrücken (6), worin jede der Strömungsbrücken (6) ausgestaltet sind, Flüssigkeit von einer Auslaßöffnung einer der mehreren Gasaustauscheinheiten (3) zu einer Einlaßöffnung einer anderen der mehreren Gasaustauscheinheiten (3), zu leiten, worin die Strömungsbrücken (6) die Gas- Strömungskanäle (4) kreuzen.

14. Gasaustauscher nach Anspruch 13, worin in jeder der Gasaustauscheinheiten (3), die Nanoröhrchen (2) Carbon-Nanoröhrchen mit einem Durchmesser zwischen 5 und 20 nm sind, worin die Vorderseite und die Rückseite zwischen 0,3 und 3 cm entfernt sind, und worin die Nanoröhrchen (2) in einer Dichte von mindestens 100 Nanoröhrchen per $\mu m^2$ gepackt sind.

**15.** Gasaustauscher nach Anspruch 13, welcher weiter umfasst:

eine erste Pumpe (76), die ausgestaltet ist, das Gas durch die Gas-Strömungskanäle (4) zu pumpen, wahlweise weiter umfassend:

eine zweite Pumpe, die ausgestaltet ist, die Flüssigkeit durch die Gasaustauscheinheiten (3) zu pumpen.

## Revendications

**1.** Unité d'échange de gaz pour traiter le sang qui comprend des globules rouges et du plasma, l'unité d'échange de gaz étant **caractérisée par** :

une enceinte étanche aux fluides ayant une face avant avec un orifice d'entrée pour faire entrer le sang, et une face arrière avec un orifice de sortie pour faire sortir le sang, l'enceinte étanche aux fluides ayant un intérieur et un extérieur ; et
un paquet de nanotubes (2) qui se situent entre la face avant et la face arrière, chacun des nanotubes (2) ayant une extrémité avant et une extrémité arrière, avec un vide (5) dans le paquet qui s'étend de l'orifice d'entrée sur la face avant à l'orifice de sortie sur la face arrière, le vide (5) étant configuré pour former un canal d'écoulement qui est assez grand pour permettre aux globules rouges de s'écouler de l'orifice d'entrée à l'orifice de sortie,

dans laquelle les nanotubes (2) du paquet sont espacés assez près les uns des autres pour retenir les globules rouges dans le canal d'écoulement, mais suffisamment éloignés pour permettre au plasma de s'écouler à travers les espaces situés entre des nanotubes attenants (2) du paquet,
dans laquelle les nanotubes (2) sont agencés par rapport à la face avant et à la face arrière pour permettre aux molécules d'$O_2$ de se diffuser dans les nanotubes (2) depuis l'extérieur de l'enceinte et pour permettre aux molécules de $CO_2$ de se diffuser à l'extérieur des nanotubes (2) jusqu'à l'extérieur de l'enceinte, et
dans laquelle les nanotubes (2) du paquet présentent des défauts dans leurs parois qui permettent aux molécules d'$O_2$ et aux molécules de $CO_2$ de se diffuser à travers celles-ci, et les défauts sont présents en un nombre et une superficie totale suffisants pour que l'unité d'échange de gaz puisse efficacement distribuer l'$O_2$ au sang et emporter le $CO_2$ loin du sang.

**2.** Unité d'échange de gaz selon la revendication 1,
dans laquelle les nanotubes (2) sont des nanotubes de carbone.

**3.** Unité d'échange de gaz selon la revendication 1,
dans laquelle la face avant et la face arrière sont espacées de 0,3 à 3 cm.

**4.** Unité d'échange de gaz selon la revendication 1, dans laquelle les nanotubes (2) ont un diamètre entre 5 et 20 nm.

**5.** Unité d'échange de gaz selon la revendication 1,
dans laquelle les nanotubes (2) du paquet, à l'extérieur du vide (5), sont tassés à une densité d'au moins 100 nanotubes par $\mu m^2$.

**6.** Unité d'échange de gaz selon la revendication 1, dans laquelle le canal d'écoulement présente une section transversale entre 250 et 2 500 $\mu m^2$.

**7.** Unité d'échange de gaz selon la revendication 1,
dans laquelle la face avant comporte une pluralité d'orifices d'entrée supplémentaires pour faire entrer le sang, la face arrière comporte une pluralité d'orifices de sortie supplémentaires pour faire sortir le sang, et le paquet de nanotubes (2) comporte une pluralité de vides supplémentaires (5) qui s'étendent des orifices d'entrée supplémentaires respectifs aux orifices de sortie supplémentaires respectifs, les vides supplémentaires (5) étant configurés pour former des canaux d'écoulement supplémentaires qui sont assez grands pour permettre aux globules rouges

EP 2 758 093 B1

de s'écouler à travers ceux-ci.

8. Unité d'échange de gaz selon la revendication 1 ou 8, dans laquelle les nanotubes sont des nanotubes de carbone ayant un diamètre entre 5 et 20 nm, la face avant et la face arrière sont espacées de 0,3 à 3 cm, les nanotubes (2) du paquet, à l'extérieur du vide (5), sont tassés à une densité d'au moins 100 nanotubes par $\mu m^2$, et le canal d'écoulement présente une section transversale entre 250 et 2 500 $\mu m^2$.

9. Echangeur de gaz pour traiter le sang qui inclut des globules rouges et du plasma, l'échangeur de gaz étant **caractérisé par** :

au moins huit unités d'échange de gaz (3), dans lequel chacune des unités d'échange de gaz (3) inclut une enceinte étanche aux fluides ayant une face avant avec un orifice d'entrée pour faire entrer le sang, une face arrière avec un orifice de sortie pour faire sortir le sang, l'enceinte étanche aux fluides ayant un intérieur et un extérieur, et
un paquet de nanotubes (2) qui se situent entre la face avant et la face arrière, chacun des nanotubes (2) ayant une extrémité avant et une extrémité arrière, avec un vide (5) dans le paquet qui s'étend de l'orifice d'entrée sur la face avant à l'orifice de sortie sur la face arrière, le vide (5) étant configuré pour former un canal d'écoulement qui est assez grand pour permettre aux globules rouges de s'écouler de l'orifice d'entrée à l'orifice de sortie,

dans lequel les nanotubes (2) du paquet sont espacés assez près les uns des autres pour retenir les globules rouges dans le canal d'écoulement, mais suffisamment éloignés pour permettre au plasma de s'écouler à travers les espaces situés entre des nanotubes attenants (2) du paquet,
dans lequel les nanotubes (2) sont agencés par rapport à la face avant et à la face arrière pour permettre aux molécules d'$O_2$ de se diffuser dans les nanotubes (2) depuis l'extérieur de l'enceinte et pour permettre aux molécules de $CO_2$ de se diffuser à l'extérieur des nanotubes (2) jusqu'à l'extérieur de l'enceinte, et
dans lequel les nanotubes (2) du paquet présentent des défauts dans leurs parois qui permettent aux molécules d'$O_2$ et aux molécules de $CO_2$ de se diffuser à travers celles-ci, et les défauts sont présents en un nombre et une superficie totale suffisants pour que l'unité d'échange de gaz (3) puisse efficacement distribuer l'$O_2$ au sang et emporter le $CO_2$ loin du sang ;
une pluralité de canaux d'écoulement de gaz (4) agencés par rapport aux unités d'échange de gaz (3) pour permettre aux molécules d'$O_2$ de se diffuser depuis les canaux d'écoulement de gaz (4) dans les nanotubes (2) dans les unités d'échange de gaz (3) et pour permettre aux molécules de $CO_2$ de se diffuser à l'extérieur des nanotubes (2) dans les unités d'échange de gaz (3) jusqu'aux canaux d'écoulement de gaz (4) ; et
au moins quatre ponts d'écoulement (6), chacun des ponts d'écoulement (6) étant configuré pour acheminer le sang depuis un orifice de sortie de l'une de la pluralité d'unités d'échange de gaz (3) jusqu'à un orifice d'entrée d'une autre de la pluralité d'unités d'échange de gaz (3), dans lequel les ponts d'écoulement (6) croisent les canaux d'écoulement de gaz (4).

10. Echangeur de gaz selon la revendication 9,
dans lequel, dans chacune des unités d'échange de gaz (3), la face avant comporte une pluralité d'orifices d'entrée supplémentaires pour faire entrer le sang, la face arrière comporte une pluralité d'orifices de sortie supplémentaires pour faire sortir le sang, et le paquet de nanotubes (2) comporte une pluralité de vides supplémentaires (5) qui s'étendent des orifices d'entrée supplémentaires respectifs aux orifices de sortie supplémentaires respectifs, les vides supplémentaires (5) étant configurés pour former des canaux d'écoulement supplémentaires qui sont assez grands pour permettre aux globules rouges de s'écouler à travers ceux-ci, et comprenant en outre :

une pluralité de ponts d'écoulement supplémentaires (6) qui connectent des orifices de sortie supplémentaires respectifs à des orifices d'entrée supplémentaires respectifs.

11. Echangeur de gaz selon la revendication 9 ou 10, dans lequel, dans chacune des unités d'échange de gaz (3), les nanotubes (2) sont des nanotubes de carbone ayant un diamètre entre 5 et 20 nm, la face avant et la face arrière sont espacées de 0,3 à 3 cm, les nanotubes (2) du paquet, à l'extérieur du vide (5), sont tassés à une densité d'au moins 100 nanotubes par $\mu m^2$, et le canal d'écoulement présente une section transversale entre 250 et 2 500 $\mu m^2$.

12. Echangeur de gaz selon la revendication 9, comprenant en outre :

une première pompe (76) configurée pour pomper au moins l'un parmi de l'air, de l'oxygène pur et de l'air

14

oxygéné à travers les canaux d'écoulement de gaz (4), comprenant éventuellement en outre : une seconde pompe configurée pour pomper le sang à travers les unités d'échange de gaz (3).

**13.** Echangeur de gaz, **caractérisé par** :

au moins huit unités d'échange de gaz (3), dans lequel chacune des unités d'échange de gaz (3) inclut une enceinte étanche aux fluides ayant une face avant avec un orifice d'entrée pour faire entrer un liquide, et une face arrière avec un orifice de sortie pour faire sortir le liquide, l'enceinte étanche aux fluides ayant un intérieur et un extérieur, et
une pluralité de nanotubes (2) qui se situent entre la face avant et la face arrière, chacun des nanotubes (2) ayant une extrémité avant et une extrémité arrière,

dans lequel les nanotubes (2) sont agencés par rapport à la face avant et à la face arrière pour permettre aux molécules de gaz de se diffuser dans les nanotubes (2) depuis l'extérieur de l'enceinte et pour permettre aux molécules de gaz de se diffuser à l'extérieur des nanotubes (2) jusqu'à l'extérieur de l'enceinte, et
dans lequel les nanotubes (2) présentent des défauts dans leurs parois qui permettent aux molécules de gaz de se diffuser à travers celles-ci, et les défauts sont présents en un nombre et une superficie totale suffisants pour que l'unité d'échange de gaz (3) puisse efficacement distribuer le gaz au liquide ;
une pluralité de canaux d'écoulement de gaz (4) agencés par rapport aux unités d'échange de gaz (3) pour permettre aux molécules de gaz de se diffuser depuis les canaux d'écoulement de gaz (4) dans les nanotubes (2) dans les unités d'échange de gaz (3) ; et
au moins quatre ponts d'écoulement (6), chacun des ponts d'écoulement (6) étant configuré pour acheminer le liquide depuis un orifice de sortie de l'une de la pluralité d'unités d'échange de gaz (3) jusqu'à un orifice d'entrée d'une autre de la pluralité d'unités d'échange de gaz (3), dans lequel les ponts d'écoulement (6) croisent les canaux d'écoulement de gaz (4).

**14.** Echangeur de gaz selon la revendication 13,
dans lequel, dans chacune des unités d'échange de gaz (3), les nanotubes (2) sont des nanotubes de carbone ayant un diamètre entre 5 et 20 nm, la face avant et la face arrière sont espacées de 0,3 à 3 cm, et les nanotubes (2) sont tassés à une densité d'au moins 100 nanotubes par $\mu m^2$.

**15.** Echangeur de gaz selon la revendication 13, comprenant en outre :

une première pompe (76) configurée pour pomper le gaz à travers les canaux d'écoulement de gaz (4), comprenant en outre éventuellement :

une seconde pompe configurée pour pomper le liquide à travers les unités d'échange de gaz (3).

FIG. 1

FIG. 2

AIR

↑

→ BLOOD

9

SEE FIG. 3B

## FIG. 3A

1    2    F2    1    1

1    F1

6    6

F3    F3

4    4

8

12    F1    6    5    6

## FIG. 3B

1

1    2

10

8

4

9

## FIG. 3C

FIG. 4A

Cross Section B - B

FIG. 4B

FIG. 5A

FIG. 5B

FIG. 5C

FIG. 6

External GE

FIG. 7A

Implanted GE

FIG. 7B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011013075 A
- WO 2011091074 A
- DE 102007010112 A
- WO 2003076702 A
- US 6667099 A

**Non-patent literature cited in the description**

- **J. LI ; C. PAPADOPOULOS ; J. M. XUA.** Highly-ordered carbon nanotube arrays for electronics applications. *Applied Physics Letters,* 1999, vol. 75, 367-369